# EUROPEAN PATENT APPLICATION

(11) **EP 2 044 945 A1**
(43) Date of publication of application: **08.04.2009**
(21) Application number: 07768313.4
(22) Date of filing: 06.07.2007
(51) Int. Cl.: A61K 31/702, A23K 1/16, A23L 1/30, A61P 1/00, A61P 37/02, A61P 37/06

(54) **IMMUNOMODULATING AGENT**

(30) Priority: 19.07.2006 JP 2006197075; 22.09.2006 JP 2006258120; 31.05.2007 JP 2007146033
(71) Applicant: Kabushiki Kaisha Hayashibara Seibutsu Kagaku Kenkyujo, Okayama-shi Okayama 700-0907 (JP)
(72) Inventor: HINO, Keiko, Okayama-shi, Okayama 700-0907 (JP); SADAKIYO, Tsuyoshi, Okayama-shi, Okayama 700-0907 (JP); TANIGUCHI, Yoshifumi, Okayama-shi, Okayama 700-0907 (JP); IWAKI, Kanso, Okayama-shi, Okayama 700-0907 (JP)
(74) Representative: Daniels, Jeffrey Nicholas
(86) International application number: PCT/JP2007/063582
(87) International publication number: WO 2008/010428

(57) **Abstract**

An object of the present invention is to provide an immunoregulatory agent, which can be continuously ingested on daily diet and has no fear of causing side effects, and a method for regulating immunity. The present invention solves the above object by providing an immunoregulatory agent comprising lactosucrose as an effective ingredient and a method for regulating immunity using lactosucrose.

## Description

### TECHNICAL FIELD

The present invention relates to the regulation of immunity, particularly, to an immunoregulatory agent comprising lactosucrose and a method for regulating immunity by using lactosucrose.

### BACKGROUND ART

Higher organisms including humans have an immune system for protecting their bodies from infection of pathogenic organisms such as bacteria, viruses, and parasites; and the production of various antibodies and cytokines is properly regulated in the living bodies. Particularly, immunoglobulin G (IgG), immunoglobulin A (IgA), immunoglobulin E (IgE), etc., are known as antibodies, and they are significantly important factors in the immune system. In recent years, oral infectious diseases such as food poisoning tend to decrease with being living environment cleaner. However, the normal levels of IgA and IgE in living bodies are reduced and their immune systems to oral infectious diseases are lowered by the clean environment because it lowers the opportunity of being stimulated by antigens. Also, antigen-specific IgE and autoantibodies are easily produced by excess reactions to allergens and autoantigens, caused by clean environments. Therefore, it is considered that moderns tend to be affected with allergic diseases such as pollinosis, food allergy, and autoimmune disease, accordingly.

In view of a method for regulating gut immunity, International Patent Publication No. WO 02/038,146 discloses that trehalose, a kind of saccharide, acts on Peyer's patch cells and exerts an activity of regulating the production of IgA and interferon-y (IFN-γ). Also, Japanese Patent Kokai No. 325,555/2002 discloses that nigerooligosacchaides prevent the lowering of immune function caused by abiotrophy. In view of a method for regulating systemic immunity, Japanese Patent Kokai No. 40,779/2003 discloses a method for preventing or treating allergic diseases by using oligosaccharides having a galactose residue bound *via* α-1,6 linkage to the non-reducing end. However, the effects of those methods are not enough.

Lactosucrose, β-D-galactosyl-(1,4)-α-D-glucosyl-(1,2)-β-D-fructoside, is , for example, as disclosed in Japanese Patent Kokai No. 27,285/91, industrially produced by a glycosyl-transferring reaction catalyzed by β-fructofuranosidase, in which β-fructofuranosidase derived from a microorganism is allowed to act on a solution containing sucrose and lactose. Recently, it has been revealed that lactosucrose shows low digestibility, bifidus-growth promoting activity, low cariogenicity, and moisture-retaining activity, and it is used in various fields such as foods, cosmetics, pharmaceuticals, etc. However, the immunoregulatory effect of lactosucrose has been hitherto unknown.

### DISCLOSURE OF INVENTION

Under these circumstances, an object of the present invention is to provide an immunoregulatory agent, which can be continuously ingested on daily diet and has no fear of side effects, and a method for regulating immunity.

In the course of extensive studies by the present inventors, a novel knowledge, that lactosucrose promotes the secretion of IgA in intestine via intestinal immune tissues such as Peyer's patch cells by oral ingestion, was obtained. Further, it was found that lactosucrose suppresses the production of IgE, which is enhanced by immunizing an allergen together with alum adjuvant. Furthermore, it was confirmed that a composition comprising lactosucrose enhances gut immunity by oral ingestion and suppresses systemic immunity such as allergy. Based on the above knowledge, the present inventors accomplished the present invention.

The present invention solves the above object by providing an immunoregulatory agent comprising lactosucrose as an effective ingredient and a method for regulating immunity of animals, comprising a step of allowing to orally ingest lactosucrose.

According to the present invention, the immunoregulatory agent of the present invention can be effectively used for preventing and treating oral infectious diseases, allergic diseases, and autoimmune diseases because it can be used readily for regulating immunity. The immunoregulatory agent of the present invention can be continuously ingested on daily diet and has no fear of side effects.

### BRIEF DESCRIPTION OF DRAWINGS

FIG. 1 shows a time course of the score for clinical symptom of nose, caused by pollinosis.
FIG. 2 shows a time course of the score for clinical symptom of eyes, caused by pollinosis.
FIG. 3 shows a time course of the score for the difficulty on daily life, caused by pollinosis.

### EXPLANATION OF SYMBOLS

◊: Score of the control group
•: Score of the test group allowed to ingest lactosucrose
↓: The start point of cedar pollen dispersal

### BEST MODE FOR CARRYING OUST THE INVENTION

The immunoregulatory agent of the present invention comprises lactosucrose as an effective ingredient. Usually, the form of lactosucrose can be arbitrarily selected from the group consisting of syrup, crystalline powder with syrup, hydrous crystal, and amorphous solid as far as it does not inhibit the effect of the present invention. Commercially available products comprising lactosucrose can be also used in the present invention. As such products comprising lactosucrose, for example, "NYU-KA OLIGO 550" and "NYU-KA OLIGO 700", saccharide compositions commercialized by Hayashibara Shoji Inc., Okayama, Japan, and "LS-90P", a saccharide composition commercialized by ENSUIKO Sugar Refining Co., Ltd., Tokyo, Japan, can be used. The content of lactosucrose in the immunoregulatory agent of the present invention is not restricted as far as it exerts immunoregulatory effect when allowed to ingest to animals including humans, and is, usually, 1 to 100% (w/w) , preferably, 10 to 100% (w/w), more preferably, 20 to 100% (w/w), on a dry solid basis.

The term "immunoregulatory effect" as referred to as in the present invention means the enhancement of gut immunity by promoting the secretion of IgA and the regulation of excess immunologic responses in systemic immunity. The immunoregulatory effect on gut immunity is exerted by activating intestinal immune tissues such as Peyer's patch cells and promoting the production of IgA or cytokines which promote the production of IgA, for example, interleukin-6 (IL-6) and transforming growth factor-β (TGF-β), in intestine. The effect on systemic immunity is exerted by suppressing the excess immunologic responses such as allergies and autoimmune diseases to systemic immunopathologically-mediated tissues such as spleen cells via the activation of regulatory T-cell.

Lactosucrose can be independently used as the immunoregulatory agent of the present invention. Further, the immunoregulatory agent of the present invention can be used in the form of a composition comprising lactosucrose such as foods and beverages, pharmaceuticals, medicated cosmetics, health foods, feeds, and baits. Ingredients acceptable to the above forms, for example, water, alcohols, starch, proteins, dietary fibers, saccharides, lipids, vitamins, minerals, flavoring agents, colorings, sweeteners, seasonings, spices, stabilizers, antioxidants, preservatives, etc. can be incorporated into the immunoregulatory agent of the present invention. Particularly, proteins such as lactoferrin, casein, collagen, soybean protein or their hydrolyzates; flavonoids such as rutin, hesperidin, quercetin, isoflavone, and their glycosides; calcium salts such as calcium lactate, calcium glycerophosphate; vitamins such as vitamin A, vitamin B₁, vitamin B₂, vitamin B₆, vitamin B₁₂, vitamin C, vitamin D, vitamin E and their derivatives; saccharides such as sucrose, maltose, trehalose, maltosyl trehalose, nigerose, isomaltose, nigerooligosaccharides, isomaltooligosaccharides, cyclic tetrasaccharides, and cyclodextrins; amino sugars such as glucosamine, galactosamine, and mannosamine; glycosaminoglycan such as hyaluronic acid, chondroitin sulfate, and heparin sulfate; sugar alcohols such as sorbitol and maltitol; hormones such as calcitonin, estrogen, and myotropic hormone; photosensitizing dyes such as *Kankoso* No.201, *Kankoso* No.301, and Kankoso No.401; plants, fungus and those extracts such as indigo plant (*Persicaria tinctoria*)*,* Japanese basil, Chinese parsley, pfaffia, *Ganocterma lucidum, Agaricus blazei* Murill*, Phellinus linteus*, kothalahimbutu, propolis, and royal jelly; and further, saccharides for promoting the growth of Bifidobacterium, powdery milk, shell powder, coral powder, honey, L-ascorbic acid 2-glucoside, etc. can be incorporated into the immunoregulatory agent of the present invention.

Since the immunoregulatory agent of the present invention enhances gut immunity by promoting the secretion of IgA as described above, it can be advantageously used for preventing or treating diseases caused by viruses such as hepatitis A virus, poliovirus, and rotavirus; bacteria such as cholera vibrio, dysentery bacillus, *Salmonella typhosa*, campylobacter, *Burkholderia pseudomallei*, *Vibrio* parahaemolyticus, Brucella, and E. coli C-157; and parasites *such as Diphyllobothrium latum, Metagonimus yokogawai, Clonochis sinensis, Echinostoma, Paragonius,* anisakis, *Gnathostoma, Angiostrongylus cantonensis,* amoeba dysenteriae, Cryptosporidium, malaria, and microfilaria. Further, since the immunoregulatory agent of the present invention suppresses the secretion of antigen-specific IgE and the excess immunologic responses to specific antigens, it can be advantageously used for preventing or treating food allergy caused by ingesting egg, milk, wheat, buckwheat, peanut, abalone, squid, salmon roe, shrimp, orange, crab, kiwi fruit, beef, walnut, salmon, mackerel, soybean, poultry, banana, mushroom, peach, yam, apple, and gelatin; pollen disease caused by pollen of cedar, Japanese cypress, orchard grass, timothy grass, alder, rye grass, *Betula platyphylla,* ragweed, *Ambrosia trifida,* tansy, Japanese hop, *Solidago altissima,* and other gramineous weed; allergy caused by house dust, metals, and chemical substances; allergic disease such as atopic dermatitis, allergic rhinitis, allergic conjunctivitis, allergic gastroenteritis, phthisis, and hives; and autoimmune diseases such as Crohn's disease, rheumatoid arthritis, systemic lupus erythematosus, systemic scleroderma, multiple myositis, mixed connective tissue disease, periarteritis nodosa, graves disease, chronic thyroiditis, myasthenia gravis, diabetes, primary biliary cirrhosis, Guillan-Barre syndrome, Sjogren's syndrome, antiphospholipid antibody syndrome, and disseminated sclerosis. Further, the immnoregulatory agent of the present invention can be advantageously used for reducing clinical symptoms and difficulties on daily life, accompanied with the above diseases. While, the immunoregulatory agent of the present invention can be sold with denoting to have activities of preventing or treating the above diseases and reducing clinical symptoms and difficulties on daily life, accompanied with the above diseases.

The method for administrating or ingesting the immunoregulatory agent of the present invention is not restricted to specific one as far as it enables lactosucrose to reach intestine, but usually, oral or tube feeding route is selected. The dose for administrating or ingesting the immunoregulatory agent of the present invention can be arbitrarily determined regarding with the method for administrating or ingesting and the species of objective animals. The dose of lactosucrose as an effective ingredient is recommended to administrate or ingest, usually, at a dose of 0.001 to 20 g/kg-body weight/day, preferably, 0.01 to 15 g/kg-body weight/day, more preferably, 0.02 to 10 g/kg-body weight/day. In the case of a dose of lower than 0.001 g/kg-body weight/day, a prescribed effect is not exerted. Also, in the case of a dose of higher than 20 g/kg-body weight/day, the effect corresponding to the dose is not exerted. In the case of humans, the maximum dose of lactosucrose without harmful effect is confirmed to be 0.6 to 0.8 g/kg-body weight. Therefore, in the case of using the dose higher than the range described above, care should be exercised because the dose causes diarrhea to human.

Usually, the immunoregulatory agent of the present invention is applied to human, but it can be applied to vertebrates having a similar immune system to human. Therefore, the immunoregulatory agent of the present invention can be incorporated into feeds or baits for domestic animals such as cattle, horse, swine, sheep, etc.; pets such as dog, cat, monkey, etc.; poultry such as fowl, duck, turkey, etc.; fishes such as red sea bream, amberjack, etc. The feeds and baits, comprising lactosucrose, can be used for preventing infectious diseases caused by virus and bacteria of domestic animals and poultry whose immunity is reduced by environmental stress such as high or low temperatures and reducing the allergic symptoms. Therefore, such feeds and baits can be used for preventing the loss of bodily strength of domestic animals or poultry and developing them efficiently. The feeds and baits exert the effect of preventing the decrease of the milk amount of cows and egg-laying rate of fowls.

The following experiments explain the embodiments of the present invention.

### Experiment 1

### Effect of lactosucrose ingestion on the regulation of IgA production in mice

An experiment for investigating the effect of lactosucrose ingestion on the enhancement of gut immunity was carried out by measuring the amount of IgA in feces of mice. Female BALB/c mice (six weeks old) were preliminary reared for one week on "AIN-93G", containing 40% (w/w) of corn starch, 20% (w/w) of casein, 13.2% (w/w) of α-corn starch, 10% (w/w) of sucrose, 7% (w/w) of soybean oil, 5% (w/w) of cellulose, 3.5% (w/w) of mineral mixture, 1% (w/w) of vitamin mixture, 0.3% (w/w) of L-cystine, and 0.0014% (w/w) of hydroquinone, which is a standard purified diet for researching the nutrition of mice and rats published by American National Institute of Nutrition in 1993. Successively, "LS-90P", a saccharide composition comprising 91.8% (w/w) , on a dry solid basis, of lactosucrose commercialized by ENSUIKO Sugar Refining Co., Ltd., Tokyo, Japan, was incorporated into the above purified diet, in which corn starch equal to the amount of the saccharide composition comprising lactosucrose had been removed, to give a lactosucrose content of 5% (w/w), and five mice/group were allowed to ingest the resulting test diet freely. Mice, allowed to ingest "AIN-93G", as the standard purified diet, were used as control. Then, fresh feces were collected from the mice at one week, two weeks, three weeks, and four weeks after starting the administration of the test diet and the control diet, and the amount of IgA in the feces was measured by ELISA using anti-IgA antibody (Mouse IgA ELISA Quantitation Kit produced by Bethyl Laboratories, Inc., Texas, USA). The amount of IgA was expressed by mg/g-wet feces and the average value of those from five mice was determined. The results are in Table 1. The percentages in parentheses in Table 1 mean relative values to those of the control at respective weeks.

**Table 1**

| Saccharide | Amount of IgA (mg/g) | | | |
|---|---|---|---|---|
| | 1 week after | 2 weeks after | 3 weeks after | 4 weeks after |
| | (8 weeks old) | (9 weeks old) | (10 weeks old) | (11 weeks old) |
| Control | 5.6±1.1 | 2.0±0.60 | 1.4±0.36 | 1.1±0.55 |
| Lactosucrose | 13±2.6 | 5.7±2.3 | 7.6±1.4 | 8.3±1.9 |
| 5% (w/w) | (232%) | (285%) | (543%) | (755%) |

From the results in Table 1, in the case of mice reared on the control purified diet, it was observed that the amount of IgA in feces was decreased as the increase of the rearing period (age in week). The reason of the phenomenon is considered that the gut immunity of mice was reduced by decreasing the amount of antigen ingested orally because the mice were reared on clean purified diet. While, in the case of mice reared on the test diet comprising lactosucrose, the amount of IgA in feces kept a high level through the rearing period even though the test diet was also clean. Accordingly, it was revealed that lactosucrose has effects of activating gut immunity and keeping the amount of the secreted IgA at a relatively high level.

### Experiment 2

### Effect of lactosucrose on the amounts of IgA and IgG in cecal content

The effect of lactosucrose on the enhancement of gut immunity was investigated by measuring the amounts of IgA and IgG in cecal content. Mice reared for four weeks on the test diet comprising 5% (w/w) of lactosucrose or the control diet, "AIN-93G", in Experiment 1 were sacrificed and the cecums were extirpated. Successively, the cecal content in each mouse was collected from the cecum and the pH, weight, the amounts of IgA and IgG were measured. The amounts of IgA and IgG were determined by ELISA using an anti-IgA antibody and that using an anti-IgG antibody (Mouse IgG ELISA Quantitation Kit produced by Bethyl Laboratories, Inc., Texas, USA), and expressed by mg/g-cecal content and ng/g-cecal content, respectively. The results are in Table 2. The percentages in parentheses in Table 2 mean the relative values to those of the control.

**Table 2**

| Saccharide | pH | Weight (mg) | IgA (mg/g) | IgG (ng/g) |
|---|---|---|---|---|
| Control | 7.80±0.23 | 0.13±0.032 | 0.57±0.15 | 120±52 |
| Lactosucrose 5% (w/w) | 6.63±0.27 | 0.22±0.051 (169%) | 5.6±1.6 (982%) | 140±54 (117%) |

As shown in Table 2, since the pH of the cecal content was lowered and the amount of cecal content was significantly increased by lactosucrose, it was suggested that lactosucrose has effects of increasing the amount of organic acids and improving the intestinal condition. Further, lactosucrose increased the amount of secreted IgA about 10-folds without effecting the amount of IgG. The results in Tables 1 and 2 indicate that lactosucrose has an effect of enhancing gut immunity.

### Experiment 3

### Effect of lactosucrose on the production of IgA, IgG and various cytokines in Peyer's patch and mesenteric lymph nodes

In order to investigate what type of immunopathologically-mediated tissue exerts the enhancing effect on gut immunity, the amounts of IgA, IgG, and various cytokines were measured. Similarly as in Experiment 1, female BAZB/c mice (six weeks old) were preliminary reared for one week on "AIN-93G", the same standard purified diet as used in Experiment 1. Successively, "LS-90P", a saccharide composition comprising 91.8% (w/w), on a dry solid basis, of lactosucrose commercialized by ENSUIKO Sugar Refining Co., Ltd., Tokyo, Japan, was incorporated into the above purified diet, in which corn starch equal to the amount of the saccharide composition comprising lactosucrose had been removed, to give a lactosucrose content of 2% (w/w) or 5% (w/w), and five mice/group were allowed to ingest the resulting test diet freely. Mice, allowed to ingest "AIN-93G", as the standard purified diet, were used as control. At four weeks after starting the administration of the test diet, Peyer's patch and intestinal lymph nodes were respectively removed from the mice and cut using a scissor. Then, the resultants were respectively treated in 0.2% (w/v) aqueous collagenase solution at 37°C for 30 min, and filtrated using a cell strainer to obtain cell suspensions. Each cell suspension was centrifuged to collect cells and the resulting cells were suspended into a 45% (v/v) solution of "PERCOLL", commercialized by GE Healthcare, to make into a cell suspension. Each resulting suspension was successively placed on a 75% (v/v) "PERCOLL" solution and centrifuged at 1,000 x g for 10 min. Cells in a boundary phase between 45% (v/v) and 75% (v/v) "PERCOLL" solution were collected and suspended in RPMI1640 medium comprising 10% FCS and 10 mM HERPES, and the cell concentration was adjusted to 1x10⁶ cells/ml. The resulting cell suspension was placed in a 24-well plate to give one ml/well each. Then, 2 µg/ml of Concanavaline A (Con A) as a stimulator to T-cell or 10 µg/ml of lipopolysaccharide (LPS) as a stimulator to B-cell and macrophage cell was admixed with the cell suspension, and then the cells were cultured at 37°C for 48 hours using 5% (v/v) carbon dioxide gas. A culture supernatant was collected from each well, and the amounts of IgA, IgG, IFN-γ, interleukin-4 (IL-4), IL-6, interleukin-10 (IL-10), and TGF-β were measured by ELISA using an anti-mouse IgA antibody, anti-mouse IgG antibody, anti-mouse IFN-γ antibody (commercialized by Pepro Tech EC. Lid.), anti-mouse IL-4 antibody (commercialized by R&D Systems), anti-mouse IL-6 antibody (commercialized by R&D Systems), anti-mouse IL-10 antibody (commercialized by R&D Systems), and anti-mouse TGF-β antibody (commercialized by Promega Co., Wisconsin, USA), and the average values of those from five mice were determined. The results for Peyer`s patch cells and intestinal lymph node cells are respectively in Tables 3 and 4. The percentages in parentheses in the tables mean a relative value to the amount of IgA, IgG, IFN-γ, IL-4, IL-6, IL-10 or TGF-β produced by Peyer's patch cells or intestinal lymph node cells from the control mice.

**Table 3**

| | Control | | Lactosucrose 2% (w/w) | | Lactosucrose 5% (w/w) | |
|---|---|---|---|---|---|---|
| | Con A | LPS | Con A | LPS | Con A | LPS |
| IgA | 1.5±0.45 | 0.88±0.24 | 1.9±0.49 | 1.9±1.8 | 4.5±2.6 | 7.0±4.0 |
| (µg/ml) | | | (127%) | (216%) | (300%) | (795%) |
| IgG | 73±14 | 45±9.1 | 110±28 | 110±90 | 270±120 | 370±150 |
| (ng/ml) | | | (151%) | (244%) | (370%) | (822%) |
| IFN-γ | 11±5.1 | 0.82±0.61 | 11±5.0 | 1.2±0.44 | 21±13 | 3.0±2.1 |
| (ng/ml) | | | (100%) | (146%) | (191%) | (366%) |
| IL-4 | 21±0.70 | 14±1.6 | 22±0.58 | 15±1.6 | 20±0.74 | 14±0.88 |
| (pg/ml) | | | (105%) | (107%) | (95%) | (100P) |
| IL-6 | 33±8.9 | 220±69 | 35±14 | 250±27 | B4±39 | 470±250 |
| (pg/ml) | | | (106%) | (114%) | (255%) | (214%) |
| IL-10 (pg/ml) | Below DL* | 210±14 | Below DL* | 260±57 (95%) | Below DL* | 160±56 (76%) |
| TGF-β | 0.30±0.23 | 1.3±0.14 | 0.44±0.24 | 1.3±0.24 | 0.66±0.13 | 1.2±0.090 |
| (ng/ml) | | | (133%) | (100%) | (220%) | (92%) |

| | | | | | | |
|---|---|---|---|---|---|---|
| *DL: Detection limit | | | | | | |

**Table 4**

| | Control | | Lactosucrose 2% (w/w) | | Lactosucrose 5% (w/w) | |
|---|---|---|---|---|---|---|
| | Con A | LPS | Con A | LPS | Con A | LPS |
| IgA | 48±26 | 41±23 | 41±15 | 38±17 | 37±31 | 29±22 |
| (µg/ml) | | | (85%) | (93%) | (77%) | (71%) |
| IgG | 1.9±1.1 | 1.3±0.28 | 1.9±1.5 | 1.5±1.0 | 1.00±0.94 | 0.79±0.79 |
| (ng/ml) | | | (100%) | (115%) | (53%) | (61%) |
| IFN-γ | 6.7±3.8 | Below DL* | 8.5±3.4 | Below DL* | 3.8±1.2 | Below DL* |
| (ng/ml) | | | (127%) | | (57%) | |
| IL-4 | 25±2.8 | Below DL* | 22±0.89 | Below DL* | 21±1.1 | Below DL* |
| (pg/ml) | | | (88%) | | (84%) | |
| IL-6 | 2.9±0.61 | 6.9±2.0 | 3.0±0.81 | 7.4±2.6 | 2.7±0.44 | 4.3±1.1 |
| (pg/ml) | | | (103%) | (107%) | (93%) | (62%) |
| IL-10 | 11±7.8 | Below DL* | 14±15 | Below DL* | 144±12 | Below DL* |
| (pg/ml) | | | (127%) | | (127%) | |

| | | | | | | |
|---|---|---|---|---|---|---|
| *DL: Detection limit | | | | | | |

As shown in Table 3, lactosucrose dose-dependently increased the production of IgA and IgG by Peyer's patch cells, in addition, promoted the production of IL-6 and TGF-β, which are cytokines capable of promoting the secretion of IgA. The results indicate that lactosucrose has an effect of promoting the secretion of IgA on Peyer's patch cells. However, as shown in Table 4, the increase of the production of IgA and various cytokines was not detected in the case of intestinal lymph node cells. Accordingly, it is considered that the gut immunity-enhancing effect of lactosucrose is mainly exerted by Peyer's patch cells.

### Experiment 4

### Effect of lactosucrose on the production of antibody in serum

In order to investigate the effect of lactosucorse on systemic immunity, antibody level in serum was measured. Similarly as in Experiment 1, female BALB/c mice (six weeks old) were preliminary reared for one week on "AIN-93G", the same standard purified diet as used in Experiment 1. Successively, "NYU-KA OLIGO 700", a saccharide composition comprising 71.4% (w/w) , on a dry solid basis, of lactosucrose commercialized by Hayashibara Shoji Inc., Okayama, Japan, was incorporated into the above purified diet, in which corn starch equal to the amount of the saccharide composition comprising lactosucrose had been removed, to give a lactosucrose content of 2% (w/w) or 5% (w/w), and five to seven mice/group were allowed to ingest the resulting test diet freely. Mice, allowed to ingest "AIN-93G", as the standard purified diet, were used as control. At 10 days after from starting the administration of the test diet, mice were sensitized to antigen by administrating 20 µg of ovalbumin (OVA) (grade V, commercialized by Sigma Corporation) as antigen and 4.5 mg of "IMJECT®", alum commercialized by Pierce ThermoScientific as adjuvant, into the peritoneal cavity. Successively, 15 days after from the first sensitization, the second sensitization was carried out by the same procedure. The blood was collected from caudal vein of mice at one, two, and three weeks after from the second sensitization, the serum was collected by centrifugation, and the amount of IgE to OVA was measured by EIA. In the cases of IgGl and IgG2a, the amount of antibody was measured using serum collected at two weeks after from the second sensitization. The IgE and IgG2a titers were measured by capture EIA and IgGl titer was measured by indirect EIA. Each titer was calculated by calibration curve prepared by using anti-OVA mouse serum (IgE: 1.760 U/ml, IgGl: 128,000 U/ml, IgG2a: 7,040 u/ml) as a standard serum. The amounts of IgE at one, two, or three weeks after from the second sensitization are in Table 5, and the amounts of IgGl and IgG2a of two weeks after from the second sensitization are in Table 6. The percentages in parentheses in tables mean a relative value to the antibody concentration in serum of the control mice.

**Table 5**

| | Anti-OVA-IgE (U/ml) | | |
|---|---|---|---|
| | Control | Lactosucrose 2% (w/w) | Lactosucrose 5% (w/w) |
| One week after the 2nd sensitization | 310±43 | 270±160 | 290±77 |
| | | (87%) | (94%) |
| two weeks after the 2nd sensitization | 260±30 | 150±35 | 120±32 |
| | | (58%) | (46%) |
| Three weeks after the 2nd sensitization | 210±38 | 110±62 | 160±57 |
| | | (52%) | (48%) |

**Table 6**

| | Control | Lactosucrose | Lactosucrose |
|---|---|---|---|
| | | 2% (w/w) | 5% (w/w) |
| Anti-OVA-IgGl | 40±18 | 27±10 | 28±7.5 |
| (x 10⁴ U/ml) | | (68%) | (70%) |
| Anti-OVA-IgG2a | 9.8±7.0 | 4.0±3.4 | 4.8±3.8 |
| (x 10⁴ U/ml) | | (41%) | (49%) |

As shown in Table 5, a large amount of anti-OVA-IgE was secreted independently of the administration of lactosucrose at one week after the second sensitization. However, comparing the data at two weeks after the second sensitization, it was revealed that anti-OVA-IgE level in serum was significantly decreased in the group administrated with lactosucrose. In addition, as shown in Table 6, IgG1 and IgG2a levels in serum were also significantly decreased in the group administrated with lactosucrose at two weeks after the second sensitization. Therefore, it is suggested that lactosucrose has an effect of suppressing the immunologic responses to the antigen (OVA) sensitized by alum adjuvant of host and decreased the anti-OVA-IgE level in serum. Accordingly, it is considered that lactosucrose suppresses systemic immunity and exerts the effect of suppressing the excess immunologic responses such as allergy.

### Experiment 5

### Effect of lactosucrose on the production of anti-Cry j 1-antibody in serum

In order to investigate the usefulness of lactosucrose ingestion for preventing or treating allergy to cedar pollen, the effect of lactosucrose for suppressing the production of IgE, caused by Cry j 1, a cedar pollen allergen, was investigated as follows. Except for using 20 µg of "PURIFIED CEDAR POLLEN ALLERGEN Cry j 1", *Cry* j 1 commercialized by Seikagaku Corporation, Tokyo, Japan, as a substitute for OVA, the experiment was carried out by the same procedure as in Experiment 4, and the amount of anti-Cry j 1-IgE was measured at one and two weeks after the second sensitization. Further, the amount of anti-Cry j 1-IgG1 was also measured at two weeks after the second sensitization. The amounts of anti-Cry j 1-IgE and anti-Cry j 1-IgG1 were measured by the same procedure as in Experiment 4 except for using "PURIFIED CEDAR POLLEN ALLERGEN *Cry* j 1-Biotin", commercialized by Seikagaku Corporation, Tokyo, Japan, as a solid phase. The results for IgE and IgG1 are in Tables 7 and 8, respectively.

**Table 7**

| | Anti-*Cry* j 1-IgE (U/ml) | | |
|---|---|---|---|
| | Control | Lactosucrose | Lactosucrose |
| | | 2% (w/w) | 5% (w/w) |
| One week after the 2nd sensitization | 802±91.4 | 351±277 | 620±157 |
| | | (44%) | (77%) |
| Two weeks after the 2nd sensitization | 1,060±281 | 239±180 | 404±114 |
| | | (23%) | (38%) |

**Table 8**

| | Control | Lactosucrose | Lactosucrose |
|---|---|---|---|
| | | 2% (w/w) | 5% (w/w) |
| Anti-Cry J 1- IgG1 (x 10⁴ U/ml) | 12.9±3.81 | 10.3±6.01 | 7.51±2.89 |
| | | (80%) | (58%) |

As is evident from the results in Table 7, the anti-*Cry* j 1-IgE level in the sera of control mice was rapidly increased by the second sensitization of Cry j 1, a cedar pollen antigen, and exceeded 800 U/ml and 1,000 u/ml after one week and two weeks, respectively. In comparison with the control group, the level of the group administrated with 2% (w/w) lactosucrose was suppressed to 44% and 23% after one week and two weeks, respectively. Although the suppressing effect observed in the group administrated with 5% (w/w) lactosucrose was lower than that of the group administrated with 2% (w/w) lactosucrose, no significant difference was observed between both groups and an apparent difference was considered to be within a range of error. Further, as is evident from the results in Table 8, immunologic response of the group administrated with lactosucrose is considered to be suppressed because the anti-*Cry* j I-IgG level in serum is decreased.

### Experiment 6

### Effect of lactosucrose on the production of cytokines in spleen

In order to investigate the effect of lactosucrose on systemic immunity, the ability for producing cytokines of spleen cells was measured. At two weeks after the second sensitization in Experiment 4, spleens were removed from the mice of the control group and the group administrated with lactosucrose and the spleen cells were adjusted to give a cell concentration of 1x10⁷/ml, and then placed in a 24-well plate. At two days or four days after adding 50 µg/ml of OVA, the amounts of IFN-γ, interleukin-2 (IL-2), IL-4, interleukin-5 (In-5), and IL-10 in the culture supernatant were determined by conventional EIAs using antibodies (commercialized by BD-Pharmingen) corresponding to those. The results are in Table 9. The percentages in parentheses in Table 9 mean relative values to the concentrations of respective cytokines produced by spleen cells from the control mice.

**Table 9**

| | Control | | Lactosucrose 5% (w/w) | |
|---|---|---|---|---|
| | 2 days after | 4 days after | 2 days after | 4 days after |
| IFN-γ | 0.97±0.37 | 3.3±0.69 | 1.1±0.19 | 2.9±0.91 |
| (IU/ml) | | | (113%) | (88%) |
| IL-2 | 370±110 | ND | 290±160 | ND |
| (pg/ml) | | | (78%) | |
| IL-4 | 30±9.1 | 98±29 | 32±10 | 62±31 |
| (pg/ml) | | | (107%) | (63%) |
| IL-5 | ND* | 1.6±0.41 | ND | 1.3±0.32 |
| (ng/ml) | | | | (81%) |
| IL-10 | 0.66±0.12 | 2.2±0.39 | 0.83±0.27 | 2.7±0.32 |
| (ng/ml) | | | (126%) | (123%) |

| | | | | |
|---|---|---|---|---|
| *ND: Not determined. | | | | |

From the results in Table 9, lactosucrose suppressed the production of IFN-γ, IL-2, IL-4, and IL-5 of mice spleen cells which had been sensitized with OVA. Therefore, it is suggested that lactosucrose has effects of suppressing both Th1 and Th2. Although the production of other cytokines tended to decrease, the amount of IL-10 was slightly increased. The results indicate that lactosucrose induces their systemic immunity to the side of suppression and the regulatory T-cell involves the immune suppression. From the above results, it is revealed that lactosucrose exerts the effect of suppressing the excess immunologic response in systemic immunity and it can be advantageously used for preventing or treating various allergic diseases and autoimmune diseases.

### Experiment 7

### Effect of lactosucrose on the induction of IgE caused by OVA

Considering the application of lactosucrose to allergic patients, mice which had been made sensitive to OVA by sensitizing with OVA were prepared and the suppressing effect of lactosucrose on the production of anti-OVA-IgE when exposed to OVA was investigated by allowing the above mice to ingest lactosucrose. Female BALB/c mice (six weeks old, five mice/group) were preliminary reared for one week on "AIN-93G", the same standard purified diet as used in Experiment 1. Successively, similarly as in Experiment 4, 20 µg of OVA, and 4.5 mg of alum were administrated to the peritoneal cavity of the mice and further reared for two weeks on the above standard diet. Then, the second sensitization of OVA and alum was carried out by the same procedure as used in the first sensitization. After two weeks, the mice were reared for further six weeks on the test diet comprising 2% (w/w) or 5% (w/w) lactosucrose, used in Experiment 1. After checking the mice being sensitive to OVA by measuring the amount of IgE, OVA and alum were administrated to them by the same procedure as used in the first and second sensitizations. After rearing two weeks on the test diet, blood was collected from respective mice and the concentrations of anti-OVA-IgE, anti-OVA-IgG1, and anti-OVA-IgG2a were determined by the same method as used in Experiment 4. The results are in Table 10.

**Table 10**

| | Control | Lactosucrose | Lactosucrose |
|---|---|---|---|
| | | 2% (w/w) | 5% (w/w) |
| Anti-OVA-IgE | 2,020±655 | 1,450±561 | 1,250±264 |
| (U/ml) | | (72%) | (62%) |
| Anti-OVA-IgG1 | 611±129 | 678±176 | 365±35.1 |
| (x 10⁴ U/ml) | | (111%) | (60%) |
| Anti-OVA-IgG2a | 10.3±2.83 | 12.4±5.02 | 5.77±1.93 |
| (x 10⁴ U/ml) | | (120%) | (56%) |

As is evident from the results in Table 10, in the case of control group, the anti-OVA-IgE level in serum was rapidly increased from about 100 U/ml to about 2,000 U/ml by the third sensitization by OVA. While, in the cases of the groups administrated with lactosucrose, the levels were suppressed to 62% and 72%. Therefore, it is revealed that lactosucrose has an effect of alleviating allergic symptoms. Further, since the levels of anti-OVA-IgG1 and anti-OVA-IgG2a were suppressed to be about 50%, it is suggested that the immunologic response of the mice was suppressed. The results indicate that the production of IgE is suppressed by administrating with lactosucrose to a patient sensitive to an allergen when exposed to the allergen, and lactosurose has an effect of alleviating the allergic symptoms.

### Experiment 8

### Suppression of clinical symptoms caused by ceder- and Japanese cypress-pollen allergy

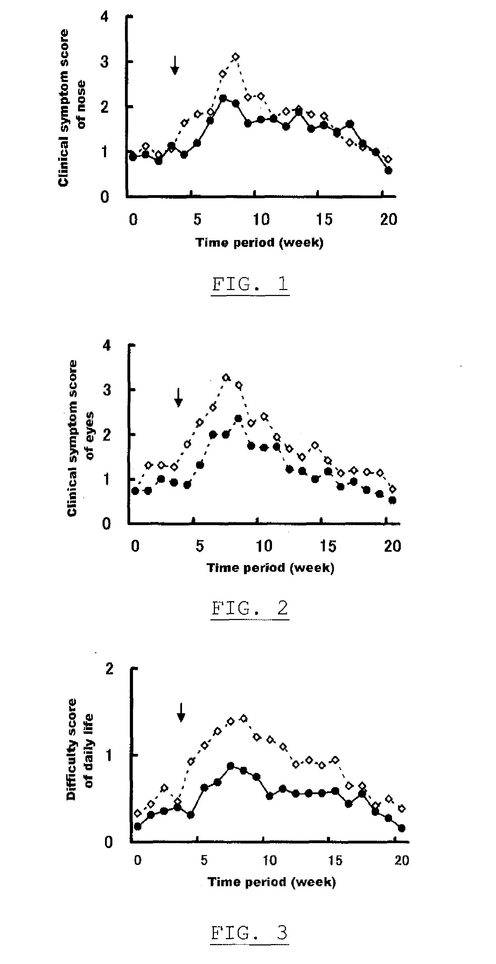

In Experiments 4 to 7, it was confirmed that the production of IgE and cytokines, which involves in allergic symptoms, is suppressed by ingesting lactosucrose. Based on the results, an experiment for investigating the suppressing effect of lactosucrose on the clinical symptoms caused by ceder- and Japanese cypress-pollen was carried out as follows. Volunteers who had been self-diagnosed to have allergic symptoms of nose or eyes at a season of cedar- and Japanese cypress-pollen (February to May) in the past two to three years, were recruited as subjects. An interview about the clinical symptoms of nose and eyes for the past two to three years was carried out to the applied volunteers with referencing a guideline for the diagnosis of pollinosis, *"*HANA ALLERGY SHINDAN GUIDELINE -TUNENSEI BIEN TO KAFUNSHO- 2005 NENDO-BAN (Guideline for the diagnosis of nose allergy -chronic allergic rhinitis and pollinosis 2005 edition)", edited by the committee for the guideline for the diagnosis of nose allergy, published by Life Science pp. 20-24 (2006). Based on the results, 40 persons estimated to be cedar- and Japanese cypress-pollen disease were selected as subjects. The subjects were divided into two groups, a test group ingesting lactosucrose (ingesting 3 g of lactosucrose every day) and a control group ingesting placebo, regarding with a score of clinical symptoms of the subjects. Then, "NYU-KA OLIGO 700", a 76% (w/w)-solid content syrup, comprising lactosucrose, commercialized by Hayashibara Shoji Inc., Okayama, Japan, which comprises 71.5% (w/w) of lactosucrose, 13.7% (w/w) of sucrose, 7.5% (w/w) of lactose, and 7.3% (w/w) of other saccharides, on a dry solid basis, was repackaged into aluminum pillowcases with about 6 g/package (3 g of lactosucrose/package) and the resulting packages were distributed to the subjects of the lactosucrose-ingesting group. In the same manner, "CORN SUGAR A-33", an about 75% (w/w)-solid content isomerized sugar syrup comprising sucrose, commercialized by Sanwa Cornstarch Co., Ltd., Nara, Japan, which comprises 33.5% (w/w) of sucrose, 33.7% (w/w) of glucose, and 29.2% (w/w) of fructose, on a dry solid basis, was repackaged into packages with about 6 g/package similarly as in the above and distributed to the subjects of the placebo-ingesting group. The ingestion was started at January 16, 2007 (zero week) , before the beginning of cedar-pollen season, and then subjects were allowed to ingest one package of the test substance at any time every day for 18 weeks. The methods for ingesting the test substance were not restricted and the subjects were permitted to ingest directly from the package or after mixing with other foods and beverages. The experiment was carried out with double blind test and the subjects were not restricted except for ingesting the test substance and were allowed to take medicines including an anti-allergic agent. For evaluating the suppression of allergic symptoms by ingesting the test substance, questionnaire survey was carried out to subjects about clinical symptoms of nose and eyes and difficulties on daily life once per week for 21 weeks, from one week before from starting the ingestion of the test substance to two weeks after of the end of the ingestion. The first questionnaire survey was carried out at the start of the ingestion (zero week). Based on the respective result of the questionnaire of the group ingesting lactosucrose and the control group, the effects were evaluated by scores determined with regarding the suppressive effect on allergic symptoms of medicines such as an anti-allergic agent used during the test period. Time course of scores for clinical symptoms of nose and eyes and difficulties on daily life are shown in FIGs. 1 to 3, respectively. According to the pollen information described on web (Ref. Pollen information website by Ministry of the Environment) , it was suggested that the start of floating cedar pollen, the peak of cedar pollen, and end of floating cedar pollen were February 9, 2007; the beginning of March; and the end of March; respectively, in the test area, the southern part of Okayama Prefecture. It was also suggested that the start of floating Japanese cypress pollen, and the end of floating Japanese cypress pollen were March 27, 2007; and the end of April in the test area. Accordingly, the ingestion of the test substance in the experiment was started about three weeks before the cedar pollen season. The results of the questionnaire were evaluated by scores according to the following methods. The health conditions of subjects were also monitored during the test period. The scores of a subject generating the clinical symptoms and difficulties on daily life caused by the reason except for pollen disease such as cold were not counted. Further, the scores of one subject in the group ingesting lactosucrose, who visited various areas except for the test area, which are different in the amount of pollen, in the test period, were not counted because the scores of the clinical symptoms and difficulties on daily life were significantly changed depending on the visiting areas.

### Method for evaluating the degree of clinical symptoms of nose and eyes by scores based on the results of questionnaire

According to the method proposed by Japanese Society of Allergology (Ref. K. Ohkubo et al., "ALLERGY NO RYOIKI (Section of Allergy)", Vol.5, No.11, pp. 1491-1499 (1998)), the results of the questionnaire as clinical symptoms of nose were analyzed based on the criteria in Table 11 and evaluated by scores based on the criteria in Table 12. As clinical symptoms of eyes, the results of questionnaire were analyzed based on the criteria in Tale 13 and evaluated by scores based on the criteria in Table 14.

### Method for evaluating the effects of drugs used during the test period by scores

The results of the questionnaire included allergic symptoms of subjects using other drugs in the test period. Therefore, in order to compare the scores reflecting the allergy-suppressing effects of the drugs, the respective drug was evaluated by scores based on the criteria in Table 15 according to a method proposed by Japanese Society of Allergology (Y. Ishida, "Biosci. Biotecnol. Biochem.", Vol.69, No.9, pp. 1652-1660 (2005)) and *"*HANAALLERGY S.H-TATDAN GUIDELINE -TUATENSEIBIEN TO KAFUNSHO- 2005 NENDO-BAN (Guideline for the diagnosis of nose allergy -chronic allergic rhinitis and pollinosis 2005 sedition)", edited by the committee for the guideline for the diagnosis of nose allergy, published by Life Science pp. 24-27 (2006).

### Method for evaluating the degree of clinical symptoms regarding the allergy-suppressing effect of drugs by scores

Scores of clinical symptoms regarding the allergy-suppressing effect of drugs were defined as a sum of the scores of clinical symptoms of nose and eyes and those of drugs.

### Method for evaluating the degree of difficulties on daily life by scores

According to the guideline, *"*HANA ALLERGY SHINDAN GUIDELINE -TUNENSEI BIEN TO KAFUNSHO- 2005 NENDO-BAN (Guideline for the diagnosis of nose allergy -chronic allergic rhinitis and pollinosis- 2005 edition)", edited by the committee for the guideline for the diagnosis of nose allergy, published by Life Science pp. 20-24 (2006), the degree of difficulties on daily life such as working, studying, house keeping, sleeping and airing were evaluated by scores based on the criteria shown in Table 16.

**Table 11**

| Symptom | Degree of symptom | | | | |
|---|---|---|---|---|---|
| | ++++ | +++ | ++ | + | - |
| Sneezing (Time/day) | 21≤ | 11-20 | 6-10 | 1-5 | 0 |
| Blowing (Time/day) | 21≤ | 11-20 | 6-10 | 1-5 | 0 |
| Nasal congestion | Completely congested all day | Strongly congested (Frequent mouth-breathing per day) | Strongly congested (Mouth-breathing in spots per day) | Congested (No mouth-breathing) | Not congested |

**Table 12**

| Degree of nasal congestion | Score | | | | |
|---|---|---|---|---|---|
| | Degree of symptom (Sneezing or Blowing)* | | | | |
| | ++++ | +++ | ++ | + | - |
| ++++ | 4 | 4 | 4 | 4 | 4 |
| +++ | 4 | 3 | 3 | 3 | 3 |
| ++ | 4 | 3 | 2 | 2 | 2 |
| + | 4 | 3 | 2 | 2 | 1 |
| - | 4 | 3 | 2 | 1 | 0 |

| | | | | | |
|---|---|---|---|---|---|
| *: Score for more severe symptom of sneezing or blowing is used. | | | | | |

**Table 13**

| Symptom | Degree of symptom | | | | |
|---|---|---|---|---|---|
| | ++++ | +++ | ++ | + | - |
| Eyes itch | More frequently than +++ | Rubbing eyes frequently | Rubbing eyes in spots | Not have to rub eyes | None |
| Teary eyes | More frequently than +++ | Wiping tears frequently | Wiping tears in spots | Not have to wipe tears | None |

**Table 14**

| Score | | | | |
|---|---|---|---|---|
| Degree of symptom (Eyes itch or Teary eyes)* | | | | |
| ++++ | +++ | ++ | + | - |
| 4 | 3 | 2 | 1 | 0 |

| | | | | |
|---|---|---|---|---|
| *: Score for more severe symptom of eyes itch or teary eyes is used. | | | | |

**Table 15**

| Drug | Score |
|---|---|
| First and second generation antihistaminic drug | 1 |
| Chemical substance-release inhibitor | 1 |
| Local vasoconstrictor drug | 1 |
| Local anticholinergic agent | 1 |
| Local steroid drug | 2 |
| Combination of oral steroid drug and antihistaminic drug | 3 |
| Specific immunotherapeutic drug (Before maintenance dose phase) | 1 |
| Specific immunotherapeutic drug (Maintenance dose phase) | 3 |

**Table 16**

| | Degree of difficulty | | | | |
|---|---|---|---|---|---|
| | ++++ | +++ | ++ | + | - |
| Difficulty on daily life* | Significantly difficult | Strongly difficult | Midpoint of +++ and + | Almost no difficulty | Less than + |
| Score | 4 | 3 | 2 | 1 | 0 |

| | | | | | |
|---|---|---|---|---|---|
| *: Difficulty in working, studying, house keeping, sleeping, airing, etc. | | | | | |

As shown in FIGs. 1 to 3, in the case of the control group, scores of clinical symptoms of nose and eyes, and difficulties on daily life increased as time advance from four weeks after the start of ingesting the test substance. The score of clinical symptoms of nose reached a peak at eight weeks after the start of ingesting the test substance, decreased to 11 weeks, then kept the level until 15 weeks, and further decreased afterward. The score of clinical symptoms of eyes reached a peak at seven weeks after the start of ingesting the test substance and then decreased. The score of difficulties on daily life reached a peak at eight weeks after the start of ingesting the test substance, decreased to 12 weeks, then kept the level until 15 weeks, and further decreased afterward. On the other hand, in the case of the group ingesting lactosucrose, any of those scores increased as time advance from five weeks after the start of ingesting the test substance. In comparison with the control group, the generation of clinical symptoms and difficulties on daily life were delayed by one week. The score of clinical symptoms of nose reached a peak at seven weeks after the start of ingesting the test substance, decreased to nine weeks, then kept the level until 17 weeks, and further decreased afterward. The score of clinical symptoms of eyes reached at a peak at eight weeks after the start of ingesting the test substance and then decreased. The score of difficulties on daily life reached a peak at seven weeks after the start of ingesting the test substance, decreased until 10 weeks, then kept the level until 17 weeks, and further decreased afterward. Comparing the scores of the control group with those of the group ingesting lactosucrose, in the case of the clinical symptoms of nose, the scores of the both groups showed no difference to three weeks after the start of ingesting the test substance, but the score of the group ingesting lactosucrose kept lower value than those of the control group during four to 10 weeks after the start of ingesting the test substance, and no difference were observed between the both groups after the 10 weeks. The scores of clinical symptoms of eyes and difficulties on daily life of the group ingesting lactosucrose were lower than those of the control group though the test period.

These results indicate that the symptoms caused by cedar pollen disease were generated just after the start of cedar pollen season (at four weeks after the start of ingesting test substance) and the ingestion of isomerized sugar syrup comprising sucrose has no effect for suppressing the symptoms caused by pollinosis. While, in the case of the group ingesting lactosucrose (the group ingesting 3 g of lactosucrose every day) , the generation of allergic symptoms after the start of pollen season was delayed and the scores of clinical symptoms and difficulties on daily life were kept to lower value through the test period including Japanese cypress pollen season, started just after the end of cedar pollen season, in comparison with the control group. The results indicate that lactosucrose can be used for reducing the clinical symptoms caused by cedar- and Japanese cypress-pollen disease and improving the difficulties on daily life. In the last half of the test period, difference of the scores of the both groups was decreased or disappeared. The reason of this is considered that the amount of Japanese cypress pollen was decreased and the clinical symptoms of nose and eyes and difficulties on daily life were lowered correspondingly in comparison with the first half of the test period. Since the scores of the subjects in this experiment was moved correlating with the start of cedar pollen season (at four weeks after the start of ingesting test substance) , the peak of the cedar pollen season (at seven weeks after) , the end of the cedar pollen season (at 11 weeks after), the start of Japanese cypress pollen season (at 11 weeks after), and the end of the Japanese cypress season (15 weeks after), it was concluded that the scores reflect the relationship between the amount of cedar and Japanese cypress pollen and the degree of allergic symptoms and difficulties on daily life.

The following examples explain the present invention in detail. However, the present invention is not restricted by them.

### Example 1

### Sweetener

To one part by weight of "LS-90P", a saccharide composition comprising lactosucrose, which contains about 90% (w/w) lactosucrose, on a dry solid basis, commercialized by ENSUIKO Sugar Refining Co., Ltd., Tokyo, Japan, 0.05 part by weight of "αG-SWEET", α-glucosyl stevioside commercialized by Toyo Sugar Refining Co., Ltd., Tokyo, Japan, was added and mixed to homogeneity, and then the mixture was made into a granular sweetener using a granule-molding machine. The product is preferably used as a low calorie sweetener for sweetening low calorie foods and beverages for obese people and diabetic patients under controlled calorie intake. Since immune functions can be regulated by orally ingesting the product, it is useful as a health food for keeping and promoting health.

### Example 2

### Health food

Two parts by weight of gum base was softened by heating and melting, and then admixed with two parts by weight of a powdery maltose, four parts by weight of sucrose, one part by weight of the sweetener obtained in Example 1, and suitable amounts of a mint flavor and coloring. Then, the mixture was kneaded by a roll and shaped by conventional methods to make into a chewing gum. The product is a chewing gum with satisfactory flavor. Since immune functions can be regulated by orally ingesting the product, it is useful as a health food for keeping and promoting health.

### Example 3

### Fluid health diet for oral use

The following ingredients were mixed to make into a fluid diet for oral use:

| | |
|---|---|
| "LS-90P", a saccharide composition | 2 parts by weight |
| comprising lactosucrose, which contains | |
| about 90% (w/w) lactosucrose, on a dry | |
| solid basis, commercialized by ENSUIKO | |
| Sugar Refining Co., Ltd., Tokyo, Japan | |
| Powdered skim milk | 43 parts by weight |
| Powdered whole milk | 12 parts by weight |
| Syrup | 41 parts by weight |
| Glucose | 3 parts by weight |
| Vitamin A | suitable amount |
| Vitamin D | suitable amount |
| Thiamin hydrochloride | suitable amount |
| Riboflavin | suitable amount |
| Pyridoxine hydrochloride | suitable amount |
| Cyanocobalamin | suitable amount |
| Choline bitartrate | suitable amount |
| Nicotinic-acid amide | suitable amount |
| Calcium pantothenate | suitable amount |
| Ascorbic acid | suitable amount |
| Tocopherol acetate | suitable amount |
| Ferrous sulfate | suitable amount |
| Calcium hydrogen phosphate | suitable amount |
| Gum arabic | suitable amount |

The product can be used for nutritional support for patients restricted to ingest usual diet by dissolving in water and allowing to ingest through orally. Further, since the product regulates immune function, it is expected to favorably recover their health.

### Example 4

### Health food

The following ingredients were mixed to homogeneity and the resulting mixture was made into an about 200 mg-tablet using a tablet machine equipped with a 6 mm-diameter pestle. Since the product has a good relish and regulates immune function by oral ingesting, it can be used for a health food for keeping and enhancing health.

| | |
|---|---|
| "NYU-KA OLIGO 550", a saccharide | 40 parts by weight |
| Composition comprising lactosucrose, | |
| which contains 55% (w/w) or higher | |
| of lactosucrose, on a dry solid basis, | |
| commercialized by Hayashibara Shoji Inc., | |
| Okayama, Japan | |
| Natural coral powder | 20 parts by weight |
| Calcium lactate | 10 parts by weight |
| Powdery yogurt | 10 parts by weight |
| Guar gum | 12 parts by weight |
| "AA2G", L-ascorbic acid 2-glucoside, | 3 parts by weight |
| commercialized by Hayashibara Shoji Inc., | |
| Okayama, Japan | |
| "αG-HESPERIDINE", glucosyl-hesperidine, | 0.5 part by weight |
| commercialized by Hayashibara Shoji Inc., | |
| Okayama, Japan | |

### Example 5

### Health food

The following ingredients were mixed and made into a cheese cracker according to conventional method.

| | |
|---|---|
| Wheat flour | 100 parts by weight |
| Fat | 9 parts by weight |
| Malt extract | 1.3 parts by weight |
| Baking soda | 0.6 part by weight |
| Cheese powder | 13 parts by weight |
| "NYU-KA OLIGO 700", a saccharide | 2 parts by weight |
| composition comprising lactosucrose, | |
| which contains about 70% (w/w) | |
| lactosucrose, on a dry solid basis, | |
| commercialized by Hayashibara Shoji Inc., | |
| Okayama, Japan | |
| Sucrose | 2 parts by weight |
| Sodium chloride | 1 part by weight |
| Diammonium carbonate | 0.6 part by weight |
| Spices | suitable amount |
| Water | 33 parts by weight |

Since the product has a good relish and regulates immune function by oral ingesting, it can be used for a health food for keeping and enhancing health.

### Example 6

### Health food

Forty parts by weight of cacao paste, 10 parts by weight of cacao butter, 20 parts by weight of sucrose, and 30 parts by weight of "NYU-KA OLIGO 700", a saccharide composition comprising lactosucrose, which contains about 70% (w/w) lactosucrose, on a dry solid basis, commercialized by Hayashibara Shoji Inc., Okayama, Japan, were mixed and lowered their granular size using a refiner. Then, the mixture was kneaded at 50°C for 2 days in a "Conche" (conching machine). During the kneading, 0.5 part by weight of lecithin was admixed with the above mixture. Successively, the resulting mixture was controlled at 31°C using a thermoregulator and pored into a mold just before the butter was hardened. Then, after removing bubbles using a vibrator, chocolate was solidified by passing through a refrigerating tunnel at 10°C. The product was removed from the mold and packaged to make into a chocolate. The product shows no hygroscopicity, but shows good color and gloss, and fine texture. The product easily melts in the mouth and shows nice sweetness and good relish. Further, since the product regulates immune function by oral ingesting, it can be used for a health food for keeping and enhancing health.

### Example 7

### Health food

According to conventional method, 100 parts by weight of yogurt, 50 parts by weight of "NYCT-KA OLIGO 550", a saccharide composition comprising lactosucrose, which contains 55% (w/w) or higher of lactosucrose, on a dry solid basis, commercialized by Hayashibara Shoji Inc., Okayama, Japan, 10 parts by weight of trehalose, 0.25 part by weight of yogurt flavor, and 0.1 part by weight of lemon essence were mixed, and then water was admixed with the mixture to give a total weight of 1,000 parts by weight to make into a yogurt drink. The product has a good relish and can be used for regulating intestinal function by controlling intestinal bacterial flora. Further, since the product regulates immune function, it can be used for a health food for keeping and enhancing health.

### Example 8

### Health food

Seventy parts by weight of "NYU-KA OLIGO LS-90P", a saccharide composition comprising lactosucrose, which contains about 90% (w/w) lactosucrose, on a dry solid basis, commercialized by ENSUIKO Sugar Refining Co., Ltd., Tokyo, Japan, 10 parts by weight of erythritol, 20 parts by weight of "HAYASHIBARA ROYAL JELLY X", a royal jelly extract commercialized by Hayashibara Biochemical Laboratories Inc., Okayama, Japan, 10 parts by weight of Coenzyme Q₁₀, 10 parts by weight of "ASCOFRESH", ascorbic acid 2-glucoside commercialized by Hayashibara Shoji Inc., Okayama, Japan, one part by weight of vitamin B₁, one part by weight of vitamin B₂, one part by weight of vitamin B₆, 0.5 part by weight of orange powder and 0.1 part by weight of lemon essence were mixed, dried, and granulated, and 2 g aliquots of which were respectively packaged into a health food. The product has a good relish and can be used for regulating intestinal function by controlling intestinal bacterial flora. Further, since the product regulates immune function, it can be used for a health food for keeping and enhancing health. The product can be sold as an immunoregulatory agent with a denotation of immunoregulatory effect.

### Example 9

### Health food

Fifty parts by weight of "NYU-KA OLIGO LS-90P", a saccharide composition comprising lactosucrose, which contains about 90% (w/w) lactosucrose, on a dry solid basis, commercialized by ENSUIKO Sugar Refining Co., Ltd., Tokyo, Japan, 30 parts by weight of rose petal extract, 3 parts by weight of "HAYASHIBARA ROYAL JELLY X", a royal jelly extract commercialized by Hayashibara Biochemical Laboratories Inc., Okayama, Japan, 3 parts by weight of an Engelhardtia *chrsolepsis* HNCE leaf extract, 3 parts by weight of a Rubus *suavissimus S.* Lee extract, 7 parts by weight of "TREHA", a hydrous crystalline α,α-trehalose commercialized by Hayashibara Shoji Inc., Okayama, Japan, 3 parts by weight of an emulsifier (sucrose-fatty acid ester) , and one part by weight of marine crude magnesium chloride were mixed, dried, and granulated, and 2 g aliquots of which were packaged into a health food. The product has a good relish and can be used for regulating intestinal function by controlling intestinal bacterial flora. Further, since the product regulates immune function, it can be used for a health food for keeping and enhancing health. The product can be sold as an immunoregulatory agent with a denotation of immunoregulatory effect.

### Example 10

### Feed for laying hen

"NYU-KA OLIGO 550", a saccharide composition comprising lactosucrose, which contains 55% (w/w) or higher of lactosucrose, on a dry solid basis, commercialized by Hayashibara Shoji Inc., Okayama, Japan, was admixed with a commercially available feed for laying hen with the following composition to give a final lactose content in the feed of 0.5% (w/w) to make into a feed comprising lactosucrose.

| | |
|---|---|
| Maize | 54.8 parts by weight |
| Soybean cake | 12.6 parts by weight |
| Rape seed cake | 3.2 parts by weight |
| Calcium carbonate | 8.5 parts by weight |
| Gluten meal | 3.5 parts by weight |
| Gluten feed | 2.0 parts by weight |
| Fish lees | 2.5 parts by weight |
| Animal fat, sodium chloride, vitamins, | 5.9 parts by weight |
| minerals, etc. | |

The product is useful as a feed for fowls. Particularly, the product enhances gut immunity of fowls, whose immunity is reduced by environmental stress such as high or low temperature. Therefore, it can be used for preventing infectious diseases caused by viruses and bacteria, and for keeping and enhancing health of fowls regardless of summer and winter. The product can be used for preventing the loss of strength and the decrease of egg-laying rate.

### INDUCTRIAL APPLICABILITY

As described above, the immunoregulatory agent of the present invention has no side effects and regulates immune function by orally-ingesting routinely. Therefore, the agent can be used for preventing and treating infectious diseases such as food poisoning, allergic symptoms, and autoimmune diseases. Further, the agent can be preferably used for health maintenance and health-promoting because it improves intestinal condition by increasing enteric bacteria such as Lactobacillus and *Bifidobacteria.*

## Claims

1. An immunoregulatory agent, comprising lactosucrose as an effective ingredient.

2. The immunoregulatory agent of claim 1, which enhances gut immunity.

3. The immunoregulatory agent of claim 2, where the enhancement of gut immunity is caused by increasing the amount of immunoglobulin A secreted in gut.

4. The immunoregulatory agent of claim 1, which suppresses systemic immunity.

5. The immunoragulatory agent of claim 4, where the suppression of systemic immunity is caused by decreasing the amount of immunoglobulin E produced in entire body.

6. The immunoregulatory agent of any one of claims 1 to 5, which is used for treating or preventing an allergic symptom.

7. The immunoregulatory agent of claim 6, wherein said allergic symptom is pollinosis or atopic dermatitis.

8. A food and beverage, pharmaceutical, feed, or bait, comprising the immunoregulatory agent of any one of claims 1 to 7.

9. The immunoregulatory agent of any one of claims 1 to 7, or the food and beverage, pharmaceutical, feed, or bait of claim 8, which is denoted as an immunoregulatory agent.

10. A method for regulating immunity of animals, said animals are allowed to orally ingest lactosucrose as an effective ingredient.
